# EUROPÄISCHE PATENTANMELDUNG

(11) **EP 0 571 795 A1**
(43) Veröffentlichungstag der Anmeldung: **01.12.1993**
(21) Anmeldenummer: 93107441.3
(22) Anmeldetag: 07.05.1993
(51) Int. Cl.: C07D 413/04, A61K 31/41

(54) **Neue 3-Phenyl-1,2,5-Oxadiazole und ihre Verwendung in der Therapie von cardiovasculären Störungen, insbesondere von Angina-Pectoris und Erektionsstörungen**

(30) Priorität: 29.05.1992 DE 4217794
(71) Anmelder: HOECHST AKTIENGESELLSCHAFT, D-65926 Frankfurt am Main (DE)
(72) Erfinder: Schönafinger, Karl, Dr., W-8755 Alzenau (DE); Bohn, Helmut, Dr., W-6369 Schöneck 1 (DE)

(57) **Zusammenfassung**

Die vorliegende Erfindung betrifft Phenylfuroxane der allgemeinem Formel I 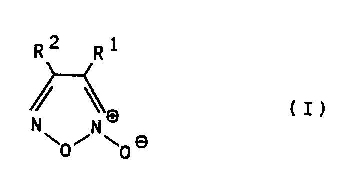
worin einer der Reste R¹ und R² für Phenyl und der andere für -S(O)ₙ-R³, -OR⁴, 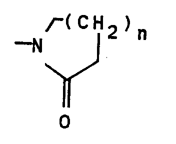
oder 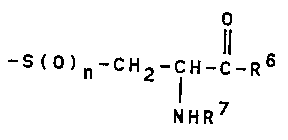
steht;
wobei R³, R⁴ sowie n wie in Anspruch 1 angegeben definiert sind, Verfahren zu ihrer Herstellung sowie ihre Verwendung.

## Beschreibung

Die vorliegende Erfindung betrifft substituierte Phenylfuroxane, Verfahren zu ihrer Herstellung und ihre Verwendung.

Eine Reihe von Verbindungen aus der Klasse der Phenylfuroxane ist bereits bekannt und beispielsweise in Helv. Chim. Acta 53 (1970), 1883 - 1892 und J. Het. Chem. 19 (1982) 2, 427 - 430 beschrieben.

Die vorliegende Erfindung betrifft Phenylfuroxane der allgemeinen Formel I 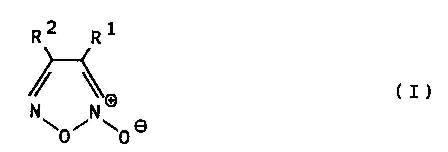
worin einer der Reste R¹ und R² für Phenyl und der andere für -S(O)ₙ-R³, -OR⁴, 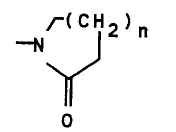
oder 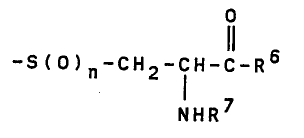
steht;
wobei
R³ (C₁-C₆)-Alkyl, (C₅-C₇)-Cycloalkyl, (C₇-C₁₀)-Aralkyl, (C₃-C₇)-Alkenyl, -(CH₂)ₘR⁵ oder -(CH₂)ₙ-CO-R⁶ bedeutet;
R⁴ (C₃-C₆)-Alkyl, (C₅-C₇)-Cycloalkyl, (C₃-C₇)-Alkenyl, -(CH₂)ₙ-Het, -(CH₂)ₘ-R⁵ oder (C₇-C₁₀)-Aralkyl bedeutet;
R⁵ Hydroxy, Alkoxy, Alkylamino, Dialkylamino oder N-Alkyl-N-aralkylamino bedeutet;
R⁶ Hydroxy, Amino, Alkylamino, Dialkylamino oder OR^{3'} bedeutet, wobei R^{3'} mit Ausnahme von -(CH₂)ₙ-CO-R⁶ wie R³ definiert ist;
R⁷ Wasserstoff, (C₁-C₆)-Alkyl oder (C₂-C₇)-Alkylcarbonyl bedeutet;
Het für einen Heterocyclus steht;
sowie
n für 0,1 oder 2 und
m für 2,3 oder 4 stehen,
sowie deren pharmakologisch annehmbaren Säureadditionsverbindungen.

Die genannten Alkylgruppen können geradkettig oder verzweigt sein. Beispiele sind Methyl, Ethyl, n-Propyl, i-Propyl, n-Butyl, i-Butyl, sek.-Butyl, tert.-Butyl, Pentyl oder Hexyl. Entsprechendes gilt für Alkoxy-, Alkylamino- oder Dialkylaminogruppen.

Für R³ oder R⁴ stehendes (C₅-C₇)-Cycloalkyl bedeutet bevorzugt Cyclopentyl und Cyclohexyl.

Für R⁵ stehendes Alkoxy ist bevorzugt (C₁-C₆)-Alkoxy. Für R⁵ und R⁶ stehendes Alkylamino oder Dialkylamino sowie für R⁵ stehendes N-Alkyl-N-aralkylamino weisen bevorzugt 1 bis 6 Kohlenstoffatome pro Alkylrest auf.

Für R⁷ stehendes (C₂-C₇)-Alkylcarbonyl ist bevorzugt Acetyl und Propionyl.

Für R³ oder R⁴ stehendes (C₇-C₁₀)-Aralkyl ist insbesondere Benzyl oder Phenylethyl.

Für R³ oder R⁴ stehendes (C₃-C₇)-Alkenyl ist insbesondere Allyl.

Für R¹ oder R² stehendes 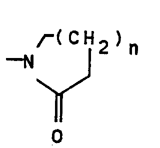
Ein für Het stehender Heterocyclus ist bevorzugt 5- bis 7-gliedrig und kann aliphatisch oder aromatisch sein. Darüberhinaus kann er auch substituiert sein. Als Heteroglieder enthält er insbesondere 〉O, 〉S oder 〉NR⁴. Sofern der Heterocyclus 2 Heteroglieder aufweist, können diese gleich oder verschieden sein. Ein Stickstoff enthaltender Heterocyclus kann auch über das Hetero-N-atom gebunden sein; er kann dann neben dem ersten, die Bindung vermittelnden Stickstoffatom noch ein beliebiges der oben genannten Keteroglieder enthalten. Beispiele für derartige, über ein Hetero-N-atom gebundene heterocyclische Reste sind der N-Pyrrolidinrest oder der N-Thiomorpholinrest.

Aromatische heterocyclische Reste sind solche, die aufgrund einer Konjugation von Doppelbindungen, gegebenenfalls mit freien Elektronenpaaren, innerhalb des Ringes mesomere Grenzstrukturen ausbilden können, wie z. B. der Thienylrest oder der Pyrasolylrest. Aliphatische heterocyclische Reste enthalten nur isolierte oder gar keine Doppelbindungen, wie z. B. der Pyrrolidinrest, der Piperidinrest, der Morpholinrest oder der Perhydrothiazepinrest. Von den zwei Heteroglieder enthaltenden Heterocyclen sind solche bevorzugt, die mindestens ein Stickstoff enthaltendes Heteroglied aufweisen.

Beispiele für Heterocyclen, von denen sich die Reste Het ableiten, sind: Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, Imidazol, Imidazolin, Imidazolidin, Oxazol, Oxazolin, Oxazolidin, Thiazol, Thiazolin, Thiazolidin, Pyrimidin, Pyridazin, Pyrazin, Piperzin, Morpholin, Thiomorpholin, Diazepin, Oxazepin, Thiazepin, Perhydrodiazepin, -oxazepin und thiazepin.

Besonders bevorzugte Reste Het leiten sich ab von Pyrrol, Pyrrolidin, Imidazol, Thiazol, Thiazolidin, Perhydrothazepin und Pyridin.

Bei den Heterogliedern der Formel 〉N-R⁴ steht R⁴ für Wasserstoff, Alkyl mit 1 bis 4, vorzugsweise 1 oder 2, C-Atomen oder Alkoxycarbonyl mit 1 bis 4 C-Atomen in der Alkokxygruppe.

Die heterocyclischen Reste können auch an einem der Ringkohlenstoffatome einen Substituenten wie z. B. eine Carboxlgruppe, eine Formylgruppe, Alkoxycarbonyl mit 1 bis 4, vorzugsweise 1 oder 2, C-Atomen in der Alkkoxygruppe oder, bevorzugt, eine Alkylgruppe mit 1 bis 4, vorzugsweise 1 oder 2, C-Atomen tragen.

Aliphatische heterocyclische Reste, insbesondere solche, die sich von Stickstoffheterocyclen ableiten, können auch an einem Ringkohlenstoffatom, vorzugsweise einem dem Stickstoff enthaltenden Heteroglied benachbarten Ringkohlenstoffatom, eine Ketofunktion, ein doppelt gebundenes Sauerstoffatom, aufweisen. Dieses kann auch in seiner tautomeren Form vorliegen.

Die heterocyclischen Reste können gegebenenfalls auch an einen Benzolkern kondensiert sein, der seinerseits gegebenenfalls durch Alkyl mit 1 bis 4, vorzugsweise 1 oder 2 C-Atomen, oder Alkoxy mit 1 bis 4, vorzugsweise 1 oder 2 C-Atomen, substituiert sein kann. Beispiele für solche kondensierten Heterocyclen sind Indol und Chinazolin.

Bevorzugte Verbindungen der allgemeinen Formel I sind solche, in denen R² Phenyl bedeutet und R¹ für -S(O)ₙ-R³ oder -OR⁴ steht.

Besonders bevorzugte Verbindungen der allgemeinen Formel I sind solche, in denen R¹ Phenyl bedeutet.

Ganz besonder bevorzugte Verbindungen der allgemeinen Formel I sind solche, in denen R¹ Phenyl und R² -S(O)ₙ-R³ oder -OR⁴ bedeutet.

Die Verbindungen der allgemeinen Formel I können beispielsweise dadurch hergestellt werden, daß eine Verbindung der allgemeinen Formel II 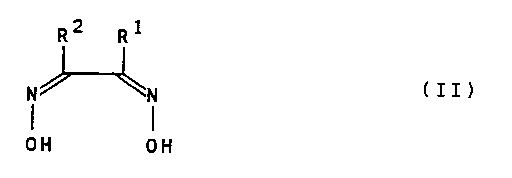
worin R¹ und R² wie oben angegeben definiert sind, oxidiert wird.

Als Oxidationsmittel können dabei herkömmliche Reagentien wie zum Beispiel Halogene, Alkalihypochlorite, Eisen-III-salze wie beispielsweise rotes Blutlaugensalz oder nitrose Gase, wie beispielsweise N₂O₄ eingesetzt werden. Die Umsetzung wird bevorzugt in einem Lösungsmittel, wie beispielsweise Wasser, einem Alkohol, einem Ether, Essigester, Methylenchlorid, Benzol, Toluol oder Chlorbenzol bei Temperaturen von -10^{o}C bis 50^{o}C, vorzugsweise von -5^{o}C bis 25^{o}C ausgeführt.

Bei der genannten Oxidation fallen in der Regel die Verbindungen der allgemeinen Formel I in Form von Isomerengemischen an. Diese lassen sich aber durch bekannte Methoden wie Umkristallisieren oder chromatographische Methoden trennen. Isomerengemische werden auch erhalten, wenn ein reines Isomeres in Substanz oder in einem inerten Lösungsmittel gelöst auf Temperaturen von 50 bis 200^{o}C erhitzt oder bei 0 bis 50^{o}C photolysiert wird. Durch Trennung des so erhaltenen Gemisches ist es somit möglich, ein Isomeres in das andere umzuwandeln.

Die Verbindungen der allgemeinen Formel II können in an sich bekannter Weise durch Nitrosierung und anschließende Umsetzung mit Hydroxylamin aus den Verbindungen der allgemeinen Formel IIa 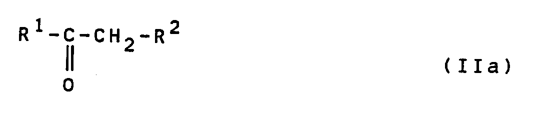
erhalten werden.

Ein bevorzugtes Verfahren zur Herstellung der Verbindungen der allgemeinen Formel I besteht darin, daß eine Verbindung der allgemeinen Formel III 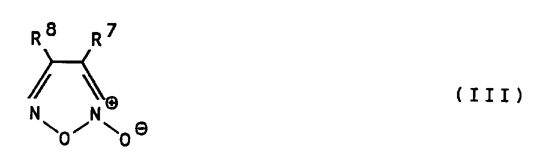
worin einer der Reste R⁷ und R⁸ für Phenyl und der andere für eine nucleofuge Gruppe steht, mit einer Verbindung HS(O)ₙ-R³, HOR⁴, 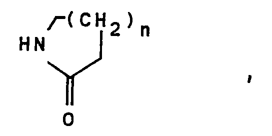
oder 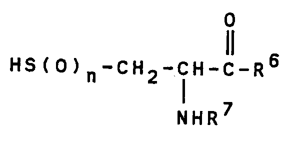
worin R³, R⁴, R⁶, R⁷ sowie n wie oben angegeben definiert sind, umgesetzt wird.

Bevorzugte nucleofuge Gruppen sind beispielsweise Halogen und insbesondere Nitro.

Vorteilhafterweise wird die Reaktion in Gegenwart einer Base ausgeführt, die die entstehenden Säuren neutralisiert. Bevorzugte Basen sind Alkalicarbonate, wie Natrium- oder Kaliumhydrogencarbonat oder Natrium- oder Kaliumcarbonat, Alkalihydroxide, wie Natrium-, Kalium- oder Lithiumhydroxid, Alkalialkoholate, wie Natrium- oder Kaliummethylat, Natrium- oder Kaliumethylat oder Kalium-tert.-butylat, Alkalihydride, wie Natrium- oder Kaliumhydrid, Alkaliamide, wie Natriumamid oder Lithiumdiisopropylamid oder organische Basen wie Pyridin oder Triethylamin. Diese Basen werden bevorzugt in molaren Mengen eingesetzt. Als Lösungsmittel kommen beispielsweise Ether, THF, Alkohole, Toluol, DMF und DMSO in Frage. Die Temperaturen liegen bei 0 bis 100^{o}C, bevorzugt 0 bis 50^{o}C.

Gegebenenfalls können die nach einem der obenstehenden Verfahren hergestellten erfindungsgemäßen Verbindungen der allgemeinen Formel I durch Modifizierung der Substituenten in weitere erfindungsgemaße Verbindungen der allgemeinen Formel I umgewandelt werden.

Beispielsweise kann die Seitenkette -SR³ mit Hilfe von Wasserstoffperoxid oder Persäuren in an sich bekannter Weise in Seitenketten -S(O)R³ oder -S(O)₂R³ überführt werden. Weiterhin können für R³ stehende Reste -(CH₂)ₙ-CO-OR^{3'} durch Reaktion mit entsprechenden Aminen in Reste -(CH₂)ₙ-CO-R⁶, worin R⁶ Amino, Alkylamino oder Dialkylamino bedeutet, oder durch Verseifung in Reste -(CH₂)ₙ-COOH umgewandelt werden.

Die Synthese der Verbindungen der allgemeinen Formel III ist an sich bekannt und beispielsweise in Izv. Akad. Nauk SSR, Ser. Khim 1990, 7, 1620 - 1622; Bull. Chem. Soc. Jpn. 63, 1843 - 1844 (1990); und in Gazz. Chim. Ital. 62 127 - 131 (1932) beschrieben.

Gemäß den vorstehenden Herstellungsverfahren können, neben den in den Beispielen beschriebenen, auch die folgenden erfindungsgemäßen Verbindungen hergestellt werden:
3-Phenyl-4-methylmercapto-1,2,5-oxadiazol-2-oxid
3-Phenyl-4-propylmercapto-1,2,5-oxadiazol-2-oxid
3-Phenyl-4-isopropylmercapto-1,2,5-oxadiazol-2-oxid
3-Phenyl-4-isobutylmercapto-1,2,5-oxadiazol-2-oxid
3-Phenyl-4-pentylmercapto-1,2,5-oxadiazol-2-oxid
3-Phenyl-4-hexylmercapto-1,2,5-oxadiazol-2-oxid
3-Phenyl-4-neopentylmercapto-1,2,5-oxadiazol-2-oxid
3-Phenyl-4-methylsulfonyl-1,2,5-oxadiazol-2-oxid
3-Phenyl-4-ethylsulfonyl-1,2,5-oxadiazol-2-oxid
3-Phenyl-4-isopropylsulfonyl-1,2,5-oxadiazol-2-oxid
3-Phenyl-4-hexylsulfonyl-1,2,5-oxadiazol-2-oxid
3-Phenyl-4-methylsulfinyl-1,2,5-oxadiazol-2-oxid
3-Phenyl-4-butylsulfinyl-1,2,5-oxadiazol-2-oxid
3-Phenyl-4-propylsulfinyl-1,2,5-oxadiazol-2-oxid
3-Phenyl-4-allylsulfinyl-1,2,5-oxadiazol-2-oxid
3-Phenyl-4-allylsulfonyl-1,2,5-oxadiazol-2-oxid
3-Phenyl-4-benzylmercapto-1,2,5-oxadiazol-2-oxid
3-Phenyl-4-phenylethylmercapto-1,2,5-oxadiazol-2-oxid
3-Phenyl-4-methoxyethylmercapto-1,2,5-oxadiazol-2-oxid
3-Phenyl-4-benzylsulfinyl-1,2,5-oxadiazol-2-oxid
3-Phenyl-4-methoxypropylsulfonyl-1,2,5-oxadiazol-2-oxid
3-Phenyl-4-benzylsulfonyl-1,2,5-oxadiazol-2-oxid
3-Phenyl-4⁻diisopropylaminoethylsulfonyl-1,2,5-oxadiazol-2-oxid
3-Phenyl-4⁻dimethylaminoethylmercapto-1,2,5-oxadiazol-2-oxid
3-Phenyl-4-isopropoxyethylsulfonyl-1,2,5-oxadiazol-2-oxid
3-Phenyl-4-cyclohexylsulfonyl-1,2,5-oxadiazol-2-oxid
3-Phenyl-4-methylaminoethylmercapto-1,2,5-oxadiazol-2-oxid
3-Phenyl-4-isopropylaminoethylsulfonyl-1,2,5-oxadiazol-2-oxid
3⁻Phenyl-4-cyclopentylsulfonyl-1,2,5-oxadiazol-2-oxid
3-Phenyl-4-phenylethoxy-1,2,5-oxadiazol-2-oxid
3-Phenyl-4-benzyloxy-1,2,5-oxadiazol-2-oxid
3-Phenyl-4-ethoxyethoxy-1,2,5-oxadiazol-2-oxid
3-Phenyl-4-methoxypropoxy-1,2,5-oxadiazol-2-oxid
3-Phenyl-4-butoxy-1,2,5-oxadiazol-2-oxid
3-Phenyl-4-cyclohexyloxy-1,2,5-oxadiazol-2-oxid
3-Phenyl-4-cyclopentoxy-1,2,5-oxadiazol-2-oxid
3-Phenyl-4-allyloxy-1,2,5-oxadiazol-2-oxid
3-Phenyl-4-methylaminoethoxy-1,2,5-oxadiazol-2-oxid
3-Phenyl-4-methoxycarbonylethylmercapto-1,2,5-oxadiazol-2-oxid
3-Phenyl-4-ethoxycarbonylethylsulfonyl-1,2,5-oxadiazol-2-oxid
3-Phenyl-4-butoxyethoxy-1,2,5-oxadiazol-2-oxid
3-Phenyl-4-(2-oxo-piperidino)-1,2,5-oxadiazol-2-oxid
sowie die entsprechenden Verbindungen, in denen die Substituenten an der 3- und 4-Position vertauscht sind, also beispielsweise 4-Phenyl-3-methylmercapto-1,2,5-oxadiazol-2-oxid usw.

Erfindungsgemäße Verbindungen der allgemeinen Formel I, die eine basische Gruppe enthalten, können mit anorganischen oder organischen Sären Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Die Verbindungen der allgemeinen Formel I und ihre pharmakologisch annehmbaren Säureadditionssalze besitzen wertvolle pharmakologische Eigenschaften. Am Modell der Kalium-depolarisierten Pulmonalarterie des Meerschweinchen führen sie in niedrigen Konzentrationen zu einer lang anhaltenden Relaxation. Diese Wirkung kann mit Oxyhämoglobin inhibiert werden, was auf einen NO-mediierten Mechanismus deutet. Stickstoffmonoxid führt als Aktivator der Guanylatcyclase zu einer Erhöhung von cyclischem Guanosinmonophosphat, welches im glatten Muskel eine Relaxation und in den Blutplättchen antiadhäsive und antiaggregatorische Wirkungen verursacht. Stickstoffmonoxid ist außerdem auch entscheidend beteiligt bei Lernvorgängen, bei der Regulation der Nierenfunktion, bei der Immunabwehr, beim septischen Schock und bei erektilen Dysfunktionen. Die erfindungsgemäßen Verbindungen können somit bei den genannten Indikationen eingesetzt werden. Vor allem aber haben sich NO-Donoren zur Behandlung und Prophylaxe von angina pectoris bewährt.

Die Verbindungen der allgemeinen Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Benzafibrat, Fenofibrat; Mittel für die Thromboseprophylaxe, wie z.B. Phenprocoumon.

Die Verbindungen der allgemeinen Formel I, ihre pharmakologisch annehmbaren Säureadditionsssalze und pharmazeutische Präparate, welche die Verbindungen der allgemeinen Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Darüberhinaus können sie auch zur Behandlung erektiler Dysfunktionen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z. B. 2 bis 4 Teilverabreichungen aufgeteilt.

### Herstellungsbeispiele

### 1. 3-Phenyl-4-(methoxycarbonyl-methylmercapto)-1,2,5-oxadiazol-2-oxid

Eine Mischung von 4,6 g 3-Phenyl-4-nitro-furoxan, 2,4 g Thioglykolsäuremethylester und 2,2 g Triethylamin in 50 ml Methanol wird unter Stickstoffatmosphäre gerührt. Dabei erwärmt sich die Mischung auf etwa 40^{o}C. Nach 1 Stunde wird auf Eiswasser gegossen, wobei sich nach kurzem Rühren ein Feststoff abscheidet, der abgesaugt und aus Isopropanol umkristallisiert wird.
Ausbeute: 3,7 g
Fp. 59 - 61^{o}C

### 2. 3-Phenyl-4-(methoxycarbonyl-methylsulfinyl)-1,2,5-oxadiazol-2-oxid

4,4 g der Verbindung aus Beispiel 1 werden in 17 ml Eisessig vorgelegt und mit 16 ml einer 35 %igen Wasserstoffperoxidlösung versetzt. Der sich abscheidende Niederschlag wird nach 2 Tagen abgesaugt, die Mutterlauge mit Eiswasser verdünnt und der sich erneut bildende Niederschlag abgesaugt. Beide Feststoffe wurden gemeinsam aus Isopropanol umkristallisiert.
Ausbeute: 3,9 g
Fp. 85 - 87^{o}C

### 3. 3-Phenyl-4-(aminocarbonyl-methylmercapto)-1,2,5-oxadiazol-2-oxid

In eine Mischung von 4 g der Verbindung des Beispiels 1 und 50 ml Methanol wird gasförmiger Ammoniak bis zur Sättigung eingeleitet. Nach 20-stündigem Stehen bei Raumtemperatur ist die Reaktion beendet. Der Niederschlag wird abgesaugt und aus Isopropanol umkristallisiert.
Ausbeute: 2,4 g
Fp. 174 - 176^{o}C

### 4. 3⁻Phenyl-4-(2-diethylamino-ethylmercapto)-1,2,5-oxadiazol-2-oxid-hydrochlorid

4,75 g 2-Diethylamino-ethylmercaptan-hydrochlorid werden in 100 ml THF unter Stickstoffatmosphäre langsam mit 3,3 g einer 50 %igen Natriumhydrid-in-Öl-Suspension versetzt. Nach 1 Stunde werden 5 g 3-Phenyl-4-nitrofuroxan eingetragen und die Mischung 20 Stunden gerührt. Die anorganischen Salze werden abgesaugt und das Filtrat eingeengt. Der Rückstand wird in Wasser aufgenommen und mit Methylenchlorid extrahiert. Nach dem Trocknen und Einengen wird der Rückstand in Essigester gelöst und das Produkt durch Einleiten von Chlorwasserstoff gefällt und abgesaugt.
Ausbeute: 4,1 g
Fp. 201^{o}C (Zers.)

### 5. 3-Phenyl-4-(2-diethylamino-ethyl-sulfonyl)-1,2,5-oxadiazol-2-oxid

2 g der Verbindung aus Beispiel 4 werden in Eisessig (15 ml) vorgelegt und mit 3,4 g 35 %igem Wasserstoffperoxid versetzt. Die Mischung wird 6 Stunden gerührt, dann eingeengt. Das zurückbleibende Öl wird mit wenig Isopropanol vermischt und mit Diisopropylether verdünnt, wobei sich ein Niederschlag abscheidet, der abgesaugt wird.
Ausbeute: 1,4 g
Fp. 136 - 138^{o}C (Zers.)

### 6. 3-Phenyl-4-(2-(N-benzyl-N-methyl-amino)-ethoxy)-1,2,5-oxadiazol-2-oxid-hydrochlorid

Eine Mischung aus 2,5 g 2-(N-Benzyl-N-methyl-amino)-ethanol, 0,7 g 50 %igem NaH, 2,5 g 3-Phenyl-4-nitrofuroxan und 30 ml THF wird 2 Stunden lang gerührt und dann eingeengt. Der Rückstand wird in Wasser aufgenommen und mit Methylenchlorid extrahiert. Nach dem Trocknen und Einrotieren wird das verbleibende Öl in Essigester gelöst und mit Chlorwasserstoff behandelt. Der sich abscheidende Niederschlag wird abgesaugt, mit Essigester gewaschen und getrocknet.
Ausbeute: 2,7 g
Fp. 163^{o}C (Zers.)
Analog den Beispielen 1 bis 6 wurden hergestellt:
7) 3-Phenyl-4-butylmercapto-1,2,5-oxadiazol-2-oxid
   Fp. - (Öl)
8) 3-Phenyl-4-(2-hydroxy-ethylmercapto-1,2,5-oxadiazol-2-oxid
   Fp. - (Öl)
9) 3-Phenyl-4-butyl-sulfonyl-1,2,5-oxadiazol-2-oxid
   Fp. 79 - 81^{o}C
10) 3-Phenyl-4-(2-hydroxy-ethyl-sulfonyl)-1,2,5-oxadiazol-2-oxid
   Fp. 57 - 60^{o}C
11) 3-Phenyl-4-(2-oxo-pyrrolidin-1-yl)-1,2,5-oxadiazol-2-oxid
   Fp. 101 - 102^{o}C
12) 3-Phenyl-4-(pyrid-3-yl-methoxy)-1,2,5-oxadiazol-2-oxid
   Fp. 110 - 112^{o}C
13) 3-Phenyl-4-(2-methoxyethoxy)-1,2,5-oxadiazol-2-oxid
   Fp. 39 - 41^{o}C
14) 3-Phenyl-4-(2-diethylaminoethoxy)-1,2,5-oxadiazol-2-oxid-hydrochlorid
   Fp. 181^{o}C (Zers.)
15) 3-Phenyl-4-(3-dimethylamino-propoxy)-1,2,5-oxadiazol-2-oxid-hydrochlorid
   Fp. 167^{o}C (Zers.)
16) S-( 3-Phenyl-2-oxo-1,2,5-oxadiazol-4-yl)-N-acetylcystein
   Fp. 159 - 161^{o}C (Zers.)
17) 3-Phenyl-4-allylmercapto-1,2,5-oxadiazol-2-oxid
   Fp. - (Öl)
18) 3-Phenyl-4-ethylmercapto-1,2,5-oxadiazol-2-oxid
   Fp. - (Öl)
19) 3-Phenyl-4-(aminocarbonyl-methylsulfinyl)-1,2,5-oxadiazol-2-oxid
   Fp. 166 - 167^{o}C
20) 3-Phenyl-4-ethylsulfonyl-1,2,5-oxadiazol-2-oxid
   Fp. 105 - 108^{o}C
21) 3-Phenyl-4-(phenylmethylmercapto)-1,2,5-oxadiazol-2-oxid
   Fp. 105 - 108^{o}C
Die pharmakologische Wirkung der Verbindungen der allgemeinen Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l CaCl₂ löst dann eine Kontraktion aus.

Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= IC₅₀, mol/l).

Folgende Werte wurden erhalten:

| Verbindung | IC₅₀ (mol/l) |
|---|---|
| 2 | 3·10⁻⁶ |
| 5 | 7·10⁻⁷ |
| 6 | 4·10⁻⁶ |
| 9 | 3·10⁻⁸ |
| 10 | 3·10⁻⁷ |
| 12 | 3·10⁻⁶ |
| 19 | 2·10⁻⁶ |
| 20 | 5·10⁻⁸ |
| Molsidomin (Vergleich) | 3·10⁻⁴ |
| Isosorbit-5-Monoitrat (Vergleich) | > 1·10⁻⁴ |

## Patentansprüche

1. Phenylfuroxane der allgemeinen Formel I 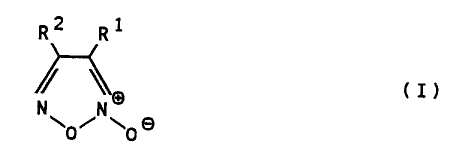 worin einer der Reste R¹ und R² für Phenyl und der andere für -S(O)ₙ-R³, -OR⁴, 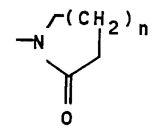 oder 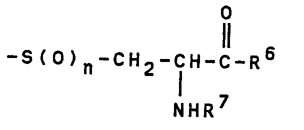 steht;
wobei
R³ (C₁-C₆)-Alkyl, (C₅-C₇)-Cycloalkyl, (C₇-C₁₀)-Aralkyl, (C₃-C₇)-Alkenyl, -(CH₂)ₘR⁵ oder -(CH₂)ₙ-CO-R⁶ bedeutet;
R⁴ (C₃-C₆)-Alkyl, (C₅-C₇)-Cycloalkyl, (C₃-C₇)-Alkenyl, -(CH₂)ₙ-Het, -(CH₂)ₘ-R⁵ oder (C₇-C₁₀)-Aralkyl bedeutet;
R⁵ Hydroxy, Alkoxy, Alkylamino, Dialkylamino oder N-Alkyl-N-aralkylamino bedeutet;
R⁶ Hydroxy, Amino, Alkylamino, Dialkylamino oder OR^{3'} bedeutet, wobei R^{3'} mit Ausnahme von -(CH₂)ₙ-CO-R⁶ wie R³ definiert ist;
R⁷ Wasserstoff, (C₁-C₆)-Alkyl oder (C₂-C₇)-Alkylcarbonyl bedeutet;
Het für einen Heterocyclus steht;
Sowie
n für 0,1 oder 2 und
m für 2,3 oder 4 stehen,
Sowie deren pharmakologisch annehmbaren Säureadditionsverbindungen.

2. Phenylfuroxane gemäß Anspruch 1, dadurch gekennzeichnet, daß R² Phenyl und R¹ -S(O)ₙ-R³ oder -OR⁴ bedeutet.

3. Phenylfuroxane gemäß Anspruch 1, dadurch gekennzeichnet, daß R¹ Phenyl bedeutet.

4. Phenylfuroxane gemäß Anspruch 1 und/oder 3, dadurch gekennzeichnet, daß R¹ Phenyl und R² -S(O)ₙ-R³ bedeutet.

5. Phenylfuroxane gemäß Anspruch 1 und/oder 3, dadurch gekennzeichnet, daß R¹ Phenyl und R² -OR⁴ bedeutet.

6. Verfahren zur Herstellung von Phenylfuroxanen der allgemeinen Formel I gemäß einem der Ansprüche 1 bis 5, dadurch gekennzeichnet, daß eine Verbindung der allgemeinen Formel III 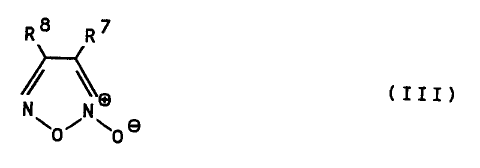 worin einer der Reste R⁷ und R⁸ für Phenyl und der andere für eine nucleofuge Gruppe steht, mit einer Verbindung HS(O)ₙ-R³, HOR⁴ oder 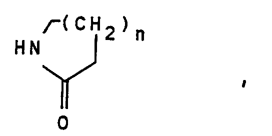 worin R³, R⁴ sowie n wie in Anspruch 1 angegeben definiert sind, umgesetzt wird.

7. Verwendung von Phenylfuroxanen der allgemeinen Formel I gemäß einem der Ansprüche 1 bis 5 zur Bekämpfung und Vorbeugung von Erkrankungen des kardiovaskulären Systems.

8. Verwendung von Phenylfuroxanen der allgemeinen Formel I gemäß einem der Ansprüche 1 bis 5 zur Bekämpfung und Vorbeugung von Angina pectoris.

9. Verwendung von Phenylfuroxanen der allgemeinen Formel I gemäß einem der Ansprüche 1 bis 5 zur Behandlung erektiler Dysfunktionen.

10. Pharmazeutisches Präparat, dadurch gekennzeichnet, daß es ein Phenylfuroxan der allgemeinen Formel I gemäß einem der Ansprüche 1 bis 5, oder ein pharmakologisch annehmbares Säureadditionssalz davon als Wirkstoff zusammen mit pharmazeutisch annehmbaren Träger- und Zusatzstoffen und gegebenenfalls noch ein oder mehrere andere pharmakologische Wirkstoffe enthält.
